# EUROPEAN PATENT SPECIFICATION

(11) **EP 4 143 896 B1**
(45) Date of publication and mention of the grant of the patent: **02.07.2025**
(21) Application number: 21717559.5
(22) Date of filing: 07.04.2021
(51) Int. Cl.: A61M 60/148, A61M 60/161, A61M 60/289, A61M 60/486, A61M 60/515, A61M 60/531, A61M 60/839, A61M 60/857, A61M 60/882, H10N 30/057, H10N 30/089, H10N 30/857

(54) **STRETCHABLE TUBULAR DEVICE AND USE THEREOF AS A COUNTERPULSATION DEVICE**
DEHNBARE RÖHRENFÖRMIGE VORRICHTUNG UND IHRE VERWENDUNG ALS GEGENPULSATIONSVORRICHTUNG
DISPOSITIF TUBULAIRE EXTENSIBLE ET SON UTILISATION COMME DISPOSITIF DE CONTREPULSATIONS

(30) Priority: 29.04.2020 EP 20171968
(43) Date of publication of application: 08.03.2023
(73) Proprietor: ECOLE POLYTECHNIQUE FEDERALE DE LAUSANNE (EPFL), 1015 Lausanne (CH)
(72) Inventor: CIVET, Yoan, 74500 Publier (FR); PERRIARD, Yves, 2000 Neuchâtel (CH); CHAVANNE, Jonathan, 1618 Châtel-St-Denis (CH); MARTINEZ, Thomas, 2000 Neuchâtel (CH); ALMANZA, Morgan, 91300 Massy (FR)
(74) Representative: P&TS SA (AG, Ltd.)
(86) International application number: PCT/IB2021/052895
(87) International publication number: WO 2021/220087

(56) References cited:
- WO-A1-03/081762
- WO-A1-2019/102417
- US-A1- 2006 217 774
- US-A1- 2008 064 917
- US-A1- 2016 144 091

## Description

### Technical domain

The present invention concerns a device adapted for cardiac assistance. In particular, the present device can be used as a implant, on an artery, and help the cardiac efforts.

### Related art

Heart failure (HF) is defined by the American Heart Association and American College of Cardiology as "a complex clinical syndrome that can result from any structural or functional cardiac disorder that impairs the ability of the ventricle to fill with or eject blood' (Hunt et al. 2009). HF is a major health problem affecting more 5.1 million patients in USA and more than 23 million worldwide (Roger 2013). HF is strongly related to age and, due to the ageing population, the prevalence is expected to rise in the future (Park and Choi 2016). Although heart transplant is a gold standard for severe heart failure, there is a need of alternative effective therapies due to the shortage of heart donors.
The first successful attempt to treat HF with an artificial assist devices in humans dates back to 1966 (DeBakey 1971). Despite the efforts of cardiac surgeons and biomedical engineers to develop and improve cardiac assist devices, the performances of these devices remain still limited. Notably, none of the devices which are commercially available are fully implantable (Sen et al. 2016, Eisen 2019)
Technological solutions to assist the heart can be divided into two big families: ventricular assist devices (VADs) (including total artificial heart, TAH) and aortic counterpulsation devices.

VADs are widely used in clinical setting as destination therapy, bridge to decision, bridge to recovery, bridge to transplantation (Loor and Gonzalez-Stawinski 2012). Current VADs are based on rotary pumps (axial and centrifugal pumps) characterized by a single rotating element which ensures a constant flow (Sen et al. 2016). The rotary pumps have replaced the pulsatile pumps of the 1990s because, compared to the latter, they are significantly simpler (lower number of parts which could fail), smaller in size (less invasive surgery required) and they are characterized by smaller/faster transcutaneous drivelines (electric rather than pneumatic) (Agarwal and High 2012). The main advantages of current VADs are associate to: i) mechanical reliability / possibility of long-term application, ii) fast response (due to electric actuation) and iii) small size (which can be advantageous for the less invasiveness of the surgery and for their potential use in fully implanted pumps). The main limitations of VADS are related to the high risk of haemolysis and thrombosis, due to the high shear generated by the rotating parts (patients must use anti-coagulant during their lifetime (Heilmann et al. 2009) and the lack of pulsatile flow. Despite the existence of conflicting findings, pulsatile flow is usually preferred to continuous flow as it favours optimal capillary perfusion (Barić 2014)

Aortic-counterpulsation devices can be subdivided in intra-aortic (de Waha et al. 2014, Santa-Cruz, Cohen and Ohman 2006), extra-aortic (Schulz et al. 2016, Davies et al. 2005, Sherwood et al. 2003) and para-aortic (Lu et al. 2011) counterpulsators depending on their location within the aorta. While intra-aortic counterpulsation (i.e. intra aortic balloon pump, IABP) has been widely used in clinical settings for the last 40 years, the applications of extra and para/aortic have been very limited. The basic principle of counterpulsation is similar for all devices: it allows an increase of the coronary blood flow (during diastole) and unloading of the left ventricle (during systole) (de Waha et al. 2014). To this end, a balloon is inflated during the diastolic phase of the heart (which translates into augmentation of the peak diastolic pressure) while presystolic deflation of the balloon decreases the afterload (the pressure against which the heart must pump during systole) (de Waha et al. 2014, Santa-Cruz et al. 2006). IABP is mainly used in high risk patients with severe left ventricular dysfunction as bridge to other solutions (e.g. VADs). The main advantages of IABPs are: i) simplicity to implant (access from femoral artery) ii) pulsatile flow is preserved and ii) anti-coagulation therapy is not required (Pucher et al. 2012). The main complications related to IABP are limb and mesenteric ischemia, bleeding and haemorrhage and infections (Pucher et al. 2012). These complications are associated to the direct arterial access (which also forces the patient to lie in bed) and the size of the pneumatic drivelines (comparing to electric lines). Moreover pneumatic actuation, mandatory for both extra and intra aortic counterpulsation devices can introduce uncontrollable delays (time for balloon inflation/deflation) and requires a huge external pneumatic systems and connection through the skin. This is incompatible with a permanent lightweight solution.

Examples of inflation balloons mentioned above and their use as counterpulsation device are given in the following documents *"*Ambulatory extra-aortic counterpulsation in patients with moderate to severe chronic heart failure", Abraham et al. JAAC, 2014, Vol 2, N°5.
"Chronic extra-aortic balloon counterpulsation : first-in-human pilot study in end-stage heart failure", Hayward et al. J. Heart Lung Transplant. 2010, 29, 1427-32. Solutions based on electroactive polymers have been investigated such as those of US20160144091. Such devices are however limited to active contractions and may not provide an optimal solution.

Further relevant prior art is for instance disclosed in documents WO2019/102417 A1, US2008/064917 A1, US2006/217774 A1 and WO03/081762 A1.

There is thus a room to develop alternative solutions, which limits or avoids the drawbacks above-mentioned. Short disclosure of the invention

An aim of the present invention is the provision of an alternative device usable as a counterpulsation device and compatible with a permanent lightweight solution.

A further aim of the present invention is the provision of a counterpulsation devices which allows to better control or eliminate potential delays in the activation of the device.

Another object of the present invention is to provide a counterpulsation device which is exempt from mechanical mobile pieces. It is in particular an object of the invention to store energy resulting from the natural artery pressure and to release energy without mobile and mechanical pieces.

It is a further object to provide a counterpulsation device which mimics the natural properties of an artery, in particular the aorta. It is more particularly an aim to provide a counterpulsation device which passively mimics the natural properties of an artery such as the aorta.

It is also an aim of the present invention to provide a method of controlling the pressure of flowing liquid using the disclosed device. In particular, the liquid is blood and the device is used to regulate the blood pressure.

The present invention further provides a process for manufacturing the disclosed device.

According to the invention, these aims are attained by the stretchable counterpulsation tubular device, the process for producing the stretchable counterpulsation tubular device and the kit described in the independent claims, and further detailed through the corresponding dependant claims.

### Short description of the drawings

Exemplar embodiments of the invention are disclosed in the description and illustrated by the following drawings :
- Figure 1a : Schematic representation of the stretchable tubular device 1 in absence of external forces. (The two layers L1, L2 are arbitrarily represented).
- Figure 1b: Schematic representation of the stretchable tubular device 1 under the application of an expending force. (only 1 layer L1 is represented for simplicity)
- Figure 1c: Schematic representation of the stretchable tubular device 1 retrieving its native diameter D0 after the expending force has decreased. (only 1 layer L1 is represented for simplicity)

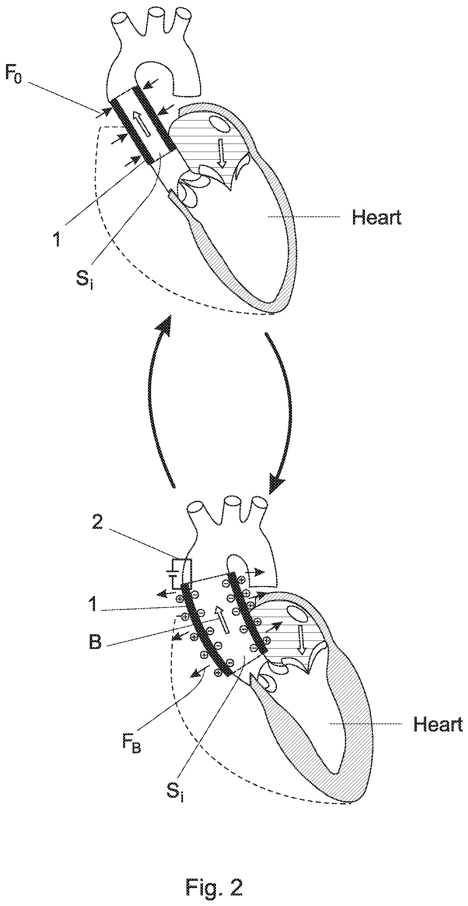

- Figure 2: Schematic view of the tubular device of the present invention used as a counterpulsation device placed on the aorta, during the systole period and during the diastole period

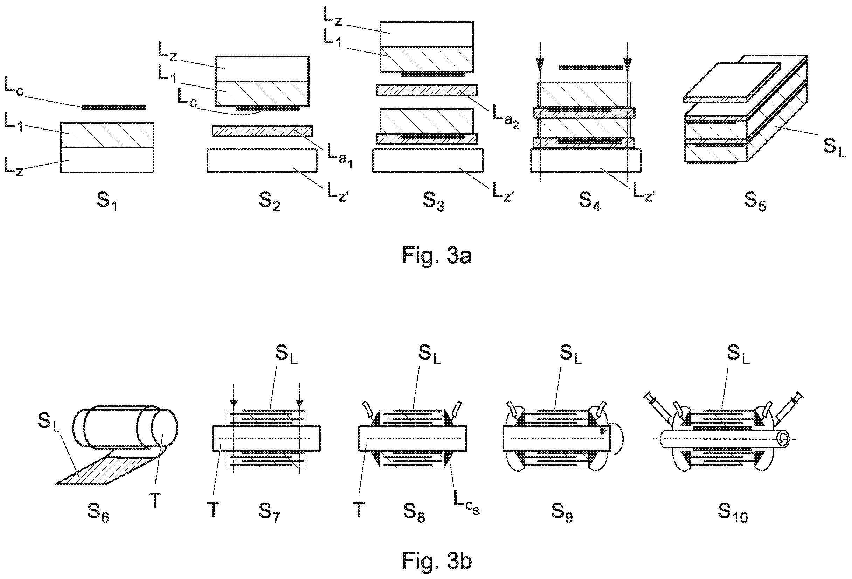

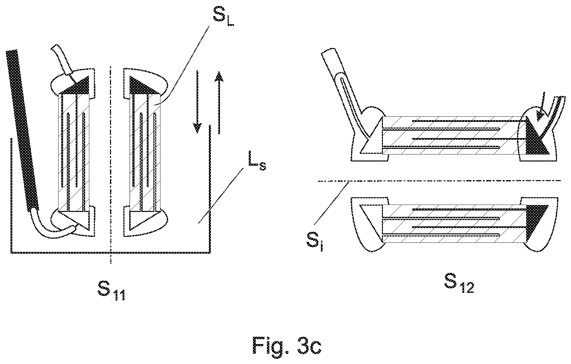

- Figures 3a, 3b, 3c : Schematic view of the process steps for the manufacturing of the stretchable device 1.

### Detailed description

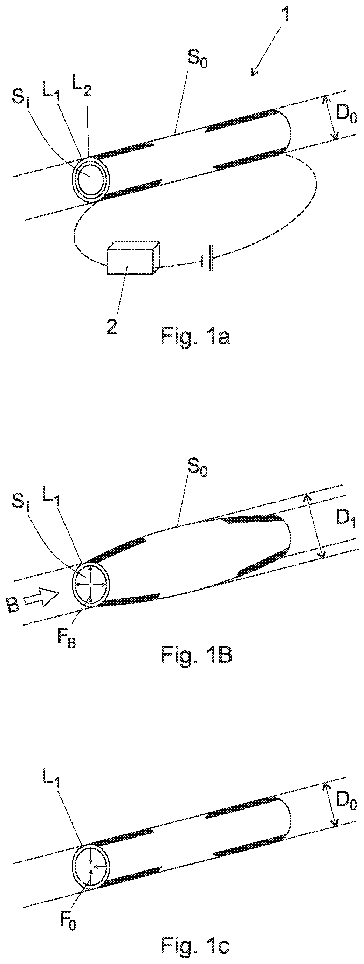

Figures 1a, 1b, 1c show a schematic view of the stretchable tubular device **1** according to the present invention. The stretchable tubular device **1** comprises one or several layers **L1, L2...Lx,** stacked in a tubular arrangement having an inner space **Si** and an outer space **So.** The inner space **Si** of the tubular device **1** may serve as a duct through which a liquid **B can** flow. The nature of the flowing liquid **B** has no importance. It can be for example pure water, an aqueous solution such as a physiological solution, an organic medium such as blood or any other liquid. The flowing liquid **B** exerts an expending force **FB** against the internal wall of the tubular device **1.** This expending force **FB,** which is schematically directed from the inner space **Si** toward the outer space **So** of the tubular device **1,** depends for example on the inner pressure of the flowing liquid **B.** The higher the inner pressure is, the stronger is the expanding force **FB** applied to the tubular device **1.** The expending force **FB** thus corresponds to the internal pressure of the tubular device **1** when a liquid **B** is flowing through. Since the tubular device **1** is stretchable, the expending force **FB** of the flowing liquid **B** tends to increase its diameter **D** above its native diameter **D0.** The native diameter **D0** should be understood here as the diameter of the tubular device **1** in absence of any external forces applied to the stretchable tubular device **1,** and in particular in absence of the expending force **FB** of the flowing liquid **B.**

The tubular device **1** has an inherent visco-elasticity **E0.** The inherent visco-elasticity **E0** should be understood as intrinsically resulting from the physical chemical properties of the layers **Lx** of the tubular device **1** in absence of any external perturbation, and in particular in absence of electrical perturbations. When the diameter **D** of the tubular device **1** is extended larger than its native diameter **D0,** then its inherent visco-elasticity exerts a natural force **F0** tending to recover its native diameter **D0.** In other words, the stretchable tubular device **1,** when undergoing the expending force **FB** of the flowing liquid **B,** opposes its natural force **F0** which reduces the expansion of its diameter **D.** The inherent visco-elasticity **E0** thus exerts a natural force **F0** directed toward the inner space **Si** of the tubular device **1** when its diameter is extended to a diameter **D1** larger than its native diameter **D0.** The natural force **F0** is thus contrary to the expending force **FB** of the flowing liquid **B.** The natural force **F0** of the tubular device **1** depends from parameters such as the length of the stretchable tubular device **1,** the nature of the stretchable layers **Lx** of polymers, their number **x,** their corresponding thickness **Tx.** The inherent visco-elasticity **E0** may be predetermined according to the application of the stretchable tubular device **1.** It may range for example between around 50 mmHg and around 200 mmH.g. It is noted here that the natural force is equivalent to a pressure since it is opposed to the internal pressure of the flowing liquid **B,** and that it can be determined with mmHg units.

Each one of the opposite faces of a given layer **Lx** is provided with an electrode or a set of electrodes **3a, 3b.** Such electrodes may for example take the form of conductive layer **Lc,** such as a carbon layer or any other known electrically conductive material. The thickness of the electrodes **Tx** may be comprised between around 10 nm and around 100 µm. It is typically in the order of around 5 to 10 µm. The electrodes may advantageously be stretchable, at least in some instance, so as to resist to the stretching of the polymer layers **Lx** to which they are connected with. The electrodes **3a, 3b** can thus induce a voltage difference between the two opposite faces of a layer **Lx.** One or several voltage level **Vn, n** being the number of voltage levels, can be applied to the stretchable tubular device **1.** At least a first level of voltage **V1** may be applied to the stretchable tubular device **1.** The application of a first voltage level **V1** modifies the visco-elasticity of the stretchable layers **Lx** of polymers, which then takes a first modified visco-elasticity **E1** value. It results that the natural force **F0** is also modified to a first modified force **F1.** When the diameter **D** of the stretchable tubular device **1** is extended above its native diameter **D0,** then the first modified force **F1,** different to the natural force **F0,** tends to recover the native diameter **D0.** As the natural force **F0,** the modified forces of the tubular device **1** are directed toward the inner space **Si** of the stretchable tubular device **1.** The first modified force **F1** is preferably lower than the natural force **F0** of the stretchable tubular device **1.** It is however not excluded that a higher force arises under the voltage application, even though this alternative is not preferred.

Under the application of a first voltage level **V1,** the first modified force **F1,** different from the natural force **F0** applies. In case the first modified force **F1** is lower than the natural force **F0,** the resistance to an expending force **FB** of a flowing liquid **B** decreases. Under a given pressure of the flowing liquid **B,** the diameter of the tubular device **1** tends to increase to a first modified diameter **D1** under the application of the first level of voltage **V1** which is higher than the diameter obtained without any voltage application. In case a second voltage **V2** is applied to the stretchable tubular device **1,** higher than the first level of voltage **V1,** then the diameter further increases to a second modified diameter **D2,** larger than the first modified diameter **D1** for the same given expending force **F0.** In other words, the stretchable tubular device **1** opposes less force to the expending force **FB** of the flowing liquid **B.** This limits the pressure increase inside the stretchable tubular device **1,** when the inner pressure of the flowing liquid **B** increases. It is here noted that the application of a voltage does not actively expends the stretchable tubular device **1,** but merely modifies its stretching properties.

The difference between the natural force **F0** and the first modified force **F1** may be determined according to the needs. For example, a difference of 3 to 20 mmHg, or 5 to 10 mmHg may be obtained when applying a first level of voltage **V1.** Such a first level of voltage **V1** may be comprised between 1000 V and 20 kV, typically between around 6kV to 12 kV.

The force of the stretchable tubular device **1,** either its natural force **F0** or one of its forces **F1, F2, Fn** modified under the application of a voltage, depends on the number **x** of layers **Lx** and their thickness **Tx.** The number of layers Lx may be comprised between 2 and 100. It may be for example around 10 to 50.

A given layer **Lx** may have a thickness **Tx** of between around 100 µm or 200 µm. A given layer Lx may have a capacitance of between around 0,8 nF and 1 nF. A given layer **Lx** may have a sheet resistance of between around 1 kΩ and 10 kΩ. The applicable voltage may vary according to the physical and chemical properties of the layers **Lx.**

According to an embodiment, a given layer **Lx** may have a thickness **Tx** of around 100 µm a capacitance of around 1,6 nF and a sheet resistance of around 1 kΩ. The first voltage level V1 may be around 6 kV. According to another embodiment, a given layer **Lx** may have a thickness **Tx** of around 200 µm, a capacitance of around 0,8 nF and a sheet resistance of around 10 kΩ. The first voltage V1 may be around 12 kV.

The electrodes **3a, 3b** are connected to a power supply **2,** which can take any usable form. In particular, the power supply may be a battery.

The change of the visco-elastic properties is immediate, meaning it takes less than 20 ms, preferably less than 1 ms under the application of a voltage.

The power supply **2** may be adapted to alternatively apply a level of voltage **V** and suppress the applied voltage **V** at predetermined frequencies. A first level of voltage **V1** may be applied for example for a duration of few milliseconds, or one second or several seconds. It may then be supressed for a dead time of few milliseconds or one second or several seconds. When the stretchable tubular device **1** surrounds a flexible duct wherein circulates a constant flow of liquid **B,** the ON/OFF alternance may induce waves of pressures. In particular, when a voltage is applied, the flowing liquid increases the diameter of the stretchable tubular device **1.** Once it is stopped, the natural force **F0** of the stretchable device 1 is recovered and the tubular device naturally retracts, generating a wave of pressure in the flowing liquid **B.**

The stretchable tubular device **1** may also be used on a none-constant flow of liquid **B,** such as the blood flow within the body. In particular, it can surround an artery or replace a portion of an artery. The present stretchable tubular device **1** may be used as an aortic counterpulsation device for example. It can store elastic energy during the systole and release it during the diastole. A first level of voltage **V1** may be applied to the stretchable tubular device **1** at the moment of the systole or shortly before the systole. Shortly before means few milliseconds to few tenth of milliseconds. Typically, a first level of voltage **V1** may be applied around 50 ms before the systole. The voltage may then be stopped at the moment of the diastole or shortly after. The recovered natural force **F0** of the stretchable tubular device **1** thus naturally retracts and releases its elastic energy, helping the heart efforts. The heart efforts may thus be assisted by more than 5%. During the systole, the decreased resistance of the tubular device **1** under the application of a voltage, limits the increase of the blood pressure, and thus limits the heart effort at this specific time. On the contrary, during the diastole, the wave of pressure is improved thanks to the natural elastic force F0, retrieved by the stretchable tubular device **1** when the applied voltage is decreased or shut off.

The level of voltage **V** may be determined according to the needs. A given stretchable tubular device **1** may be adapted for several different conditions. Depending on the necessary force modification **F1** during the systole, a predetermined first level of voltage **V1** may be determined. Alternatively, the modified force may be adaptable between a first modified force **F1** and a second modified force **F2** depending on the instant patient needs. A third modified force **F3** may equally be determined. This also allows to tune the effect of the stretchable tubular device **1** once it is implanted in the body. Also, the duration of the voltage application may be object to fine adaptation.

The applied voltage may be, alternatively or in addition, instantaneously adapted to the actual need of the patent. Depending of the physical activity of the patient, or the cardiac rhythm, or several other physiological parameters and any combination thereof, a first **V1** or a second **V2** or a third **V3** level of voltage may be instantaneously applied. For example, a maximal voltage level may be applied so as to decrease the force of the stretchable tubular device **1** to zero, during the systole. In this case, there is no increase of blood pressure and thus no additional effort from the heart. The voltage may be completely shut off at the diastole period so as to provide a maximal elastic force from the stretchable tubular device **1.** An intermediate voltage may alternatively apply during the diastole so as to not deliver the maximal elastic force from the stretchable tubular device **1.** Any combination of voltage may thus be determined. When used as such, the power supply **2** may be connected to a control unit (not shown) adapted to instantaneously determine the suitable voltage level to apply to the stretchable tubular device **1.** Sensors may also be used to sense one or several physiological parameters related to the actual heart activity. Pressure sensors may alternatively or in addition be used for determining the pressure of the liquid B flowing through the stretchable tubular device **1.**

Thus, the present invention allows for a method of helping the heart effort, in particular when the heart effort should be minimized during the systole period. The method using the stretchable tubular device further helps increasing the blood pressure at the diastole moment, by releasing the natural elastic energy of the stretchable tubular device **1.**

An ensemble or a kit comprising several different stretchable devices 1 may also be used. As an example, a kit may comprise a first stretchable tubular device (1) having a natural force (F0) and at least a first modified force (F1) and at least a second stretchable tubular device (1') having a natural force (F0') and at least a first modified force (F1'). One or more of the natural force (F0') and the modified force (F1') of the second stretchable tubular device (1') may differ from the natural force (F0) and/or the modified force (F1) of the first stretchable tubular device (1). Depending on the actual needs a particular stretchable tubular device of the kit may be selected according to its elastic properties.

Such a stretchable tubular device **1** may be used elsewhere than the aorta. A kit comprising several devices **1** may provide a first stretchable tubular device **1** adapted for the aorta, close to the heart. It may in addition offer at least one other stretchable tubular device having different properties, and dedicated to another site, or another artery.

The present disclosure also encompasses a method of regulating the pressure of a flowing liquid B. When flowing through the stretchable tubular device 1, the pressure of the flowing liquid B may be instantaneously adapted and regulated by the application of one or more different voltages to the stretchable tubular device 1. The method of regulating the pressure of a flowing liquid B can be applied to regulate the blood pressure. The stretchable tubular device 1 may thus be used as a counterpulsation device. To this end, it may be arranged on the aorta, and activated according to the following method, comprising the steps of :
- Determining a first instant **t1** on which a first level of voltage **V1** is applied to the stretchable device **1,** so as to induce a first modified force **F1,** lower than its natural force **F0.**
The first level of voltage **V1** may be either the maximal applicable voltage, adapted to reduce the force of the stretchable tubular device **1** to zero. It can alternatively be an intermediate voltage adapted to partly reduce the force of the stretchable tubular device **1.** The first instant **t1** may be predetermined or determined based on one or more sensor activation. To this end a sensor may be used to determine the instantaneous blood pressure or the instantaneous cardiac contraction. The first instant **t1** can for example correspond to the beginning of the systole period, or correspond to a moment before the systole period.

The method of regulating the pressure of a flowing liquid **B** may further comprise a second step of determining a second instant **t2** on which a second level of voltage **V2** is applied to the stretchable tubular device **1,** so as to induce a second modified force **F2.** The second instant **t2** may correspond to the beginning of the diastole period or to a moment shortly before the diastole starts or to a moment shortly after the diastole starts. The second instant **t2** may be predetermined or determined according to instantaneous sensor data allowing to determine the instantaneous blood pressure or the instantaneous cardiac contractions. The second level of voltage **V2** may be lower than the first level of voltage **V1.** It can also be completely null, in a way that the stretchable tubular device **1** retrieves its natural force F0 and provide a maximal elastic force.

The method of regulating the pressure of a flowing liquid **B** thus comprises at least two phases, a first phase starting at the first instant **t1** wherein a first voltage **V1** is applied, and ending at the beginning of the second phase, and a second phase starting at the second instant **t2,** wherein a second voltage **V2** is applied. The second phase may end at the beginning of the first phase so as to repeat and alternate the first and the second phases. One or both of these two phases may comprise the application of one or more intermediate voltages.

According to a specific embodiment, the method of use of the stretchable tubular device **1** comprises a first phase starting at the first instant **t1** wherein two intermediate voltages **V1** and **V1'** are alternatively applied several times so as to produce a fast pulsation during this first phase, and a second phase starting at the second instant **t2** wherein a constant voltage **V2** is applied. In particular, an alternance of two different voltages may be provided during the systole and the voltage may be switched off at the beginning of the diastole.

According to another embodiment, the method of use of the stretchable tubular device **1** comprises a first phase starting at the first instant **t1** wherein a first constant voltage **V1** is applied during a period shorter than the duration of the first phase, after which at least one intermediate constant voltage **V1'** is applied before the end of the first phase. Said second constant voltage may last until the end of the first phase, after the second instant **t2.** Such a constant intermediate voltage **V1'** allows to regulate the stretching of the device **1** when the blood pressure increases. In addition or alternatively, several different intermediate voltages may be successively applied during the first phase, until a final value, so as to progressively regulate the stretching of the stretchable tubular device **1** when the blood pressure decreases. The voltage may be switched off at the second instant **t2** or at another moment during the second phase. Different timing and voltage combinations may be considered depending on the needs.

The layers **Lx** of the stretchable tubular polymer **1** may comprise any electroactive polymer or mixture of electroactive polymer. For a specific medical implant application, the layers **Lx** is preferably constituted of bio-compatible polymers such as silicone or other known bio-compatible polymers. Additional layers than those of stretchable polymer may be included in the stretchable device **1.** For example, adhesion layers La may be used to maintain the stack of layers **Lx** and the corresponding electrodes **3a, 3b** together. The arrangement of the layers comprised in the stretchable tubular device **1** is open to a variety and optimisation known to one skilled in the art. The layers **Lx** of the stretchable tubular device **1** may be for example as described in the patent application CH20190001382, without being limited to its disclosure.

The stretchable tubular device **1** may in addition comprise one or more than one shield layer **Ls,** having an electrical shield effect so as to avoid or limit potential leakage of current. This may be particularly important in case the stretchable tubular device **1** is used as an implant. The shield layer **Ls** is preferably arranged on top of the layers **Lx,** so as to isolate the stack of layers **Lx** from the outer space **So** of the stretchable tubular device **1.** The shield layer **Ls** may be a conductive layer such as a carbon layer or any other conductive material suitable for an implant.

Although the stretchable tubular device 1 may be connected or may comprise one or more sensor adapted to determine some physiological parameters, such as the blood pressure, the presently disclosed device 1 can also be, alternatively or in addition, used as a sensor of such physiological parameters. A self-sensing function thus allows to better control the device and its instantaneous command.

The stretchable tubular device 1 may be produced according to the following sequence of steps.
- S1: Depositing a conductive layer **Lc** on the top surface of the first layer **L1.** Such a deposited conductive layer may be a carbon layer, deposited by known methods such as vapor deposition or coating. The conductive layer **Lc** may alternatively be a conductive gel or any other suitable material. The first layer **L1** may be free or combined with a first support layer **Lz** on its surface opposite to the top surface provided with the conductive layer **Lc.** The first support **Lz** may be made of PET or with other suitable polymer. The first layer may be for example of the type of Elastosil. The combination of the first layer **L1** and the conductive layer **Lc,** and possibly the first support layer **Lz** forms a stack.
- S2: Placing an adhesion film **La1** on a second support layer **Lz'.** The second support layer **Lz'** may be in the same material as the first support layer **Lz** or in a different material. It is preferably made in PET. Such adhesion layer **La1** may be a custom-made carbone based layer using LSR4305.
- S3 : Placing the stack of layers obtained in the step S1 on the top of the adhesion layer **La1** arranged in step S2, wherein the stack of layers is placed up-side down, meaning that the conductive layer **Lc** of the stack of layers is placed in contact with the adhesion layer **La1.** The layers are thus stacked so as to alternate the conductive layers **Lc** and the stretchable layers **Lx.**
- The previous steps S1, S2 and S3 are iterated as many times as necessary, until the desired number of layers **Lx** is obtained. To this end, another adhesion layer **La2** can be placed on the top of the stack of layers resulting from the previous sequence step S1, S2 and S3. In case a support layer **Lz** is used, it is removed before placing such adhesion layer **La2** so that it is in direct contact with the surface of the stretchable layer **L1.** A new stack of layers is organized according to step S1 and then placed up-side down on the newly added conductive layer **La2.**

Once the number of layers **Lx** is adequate, a further step S4 of cutting the edges of the stacked layers is provided and a step S5 of a final deposition of a silicon layer so as to glue the stacked layers. Before the final deposition is made, the second support layer **Lz'** may be removed. This step S5 results in a final stack of layer SL. The contact of the layers glued during step S5 will then be maintained during the following steps and in particular during the rolling step S6. The resulting final stack of layers **SL** of step S5 may have a parallelepiped shape having a longitudinal dimension comprised between around 5 to 30 cm, or 10 to 20 cm, and a transversal dimension comprised between around 2 and 10 cm, or between 3 and 5 cm.

In a step S6, the final stack of layers **SL** previously obtained is rolled around a tube **T** and the edges of the tube T are cut in a step S7 to allow electrical connection between the conductive layers and an external electrical device. The tube **T** may have a diameter comprised between 25 and 35 mm. It may for example be of around 30 mm diameter, corresponding to the diameter of the aorta at rest. The length of the tube **T** may be comprised between 30 and 80 mm, preferably between 50 and 70 mm. The tube **T** may be made or comprise a bio compatible polymer. It may be for example in PMMA. In a step S8, a layer of conductive silicone **Lcs** is deposited in order to establish the electrical contact with all the electrodes **3a, 3b** and a wire is introduced. In further steps S9 and S10, one or more of inner and outer insulation layer Li is provided. The sequence of the steps S6 to S10 allows to provide the tubular shape of the device **1.** After this sequence of steps or at any time in between, the adhesion of the layers, in the final stack of layers **SL,** may be checked by microscopic analysis.

Once the tubular shape is formed and isolated, a further step S11 of depositing a shielding layer Ls is operated by deep coating. The tubular shape may be immersed to this end in a reservoir of carbon ink. Then the necessary electrical connections are provided in a step S12. These electrical connexions include the connexion toward a power supply. This also includes the connexion of the shielding layer Ls to the ground.

It is highlighted that although the flowing liquid B described above preferably denotes the blood, it may denote any other liquid flowing through a tubular structure. The stretchable tubular device 1 is thus adapted to any system comprising a pump which pulses a liquid through a tubular structure. The stretchable tubular device 1 takes benefits of any increase of pressure of the flowing liquid, which is pulsed by a pump, and provides an additional contractile force due to the variation of its viscoelasticity. When the liquid is blood, the corresponding pump is the heart.

It is further highlighted that the voltage applied to the stretchable tubular device 1 has no direct incidence or no substantial direct incidence on its diameter. In other words, the applied voltage modifies the viscoelasticity of the stretchable tubular device without modifying or substantially modifying its diameter.

The modulation of the force applied by the stretchable tubular device exclusively depends on the voltage applied to it. In other words, the compression force provided by the stretchable tubular device 1 does not involve any additional mechanical elements such as springs or balloon.

It is thus understood that the stretchable tubular device 1 here described does not comprise additional mechanical parts such as spring or balloon. The variation of its diameter is exclusively due to the variation of pressure of the liquid flowing through it. Although it is preferably used without additional mechanical part, it may be combined with one or more of such mechanical parts allowing to actively modify its diameter. In this specific case, only its viscoelastic properties are modulated under application of a voltage, as described above.

## Claims

1. Stretchable counterpulsation tubular device (1) adapted for passively mimicking the natural properties of an artery through which blood (B) flows, comprising at least one layer (Lx) of a stretchable polymer, a power supply (2) and a set of electrodes (3a, 3b) connected to said power supply (2), wherein the power supply is adapted to supply at least a first level of voltage (V1) to said electrodes, **characterized in that** said tubular counterpulsation device has a native diameter (D0) and an inherent visco-elasticity (E0) exerting a natural force (F0) directed toward its inner space (Si) when its diameter is extended to a diameter (D1) by the expending force of the blood larger than its native diameter (D0), and **in that** it has at least a first level of modified visco-elasticity (E1) when a first level of voltage (V1) is supplied to the layers (Lx) of stretchable polymer through the electrodes (3a, 3b), said first level of modified visco-elasticity exerting a first modified force (F1) directed toward the inner space of the tubular device when its diameter is extended to a diameter (D1) by the expending force of the blood larger than its native diameter (D0), the first modified force (F1) being lower than the natural force (F0).

2. Stretchable counterpulsation tubular device according to claim 1 wherein said at least one first voltage (V1) has no incidence on the diameter of the stretchable tubular device.

3. Stretchable counterpulsation tubular device according to one of claims 1 and 2, wherein said Stretchable counterpulsation tubular device is exempt from mechanical parts such as springs and balloons.

4. Stretchable counterpulsation tubular device according to claims 1 to 3, wherein the thickness (Tx) of the at least one layer (Lx) is comprised between around 10 µm and around 500 µm, such as around 50 µm, 100 µm, 200 µm or 300 µm.

5. Stretchable counterpulsation tubular device according to claim 1 to 4, wherein the electrodes (3a, 3b) are made with carbon, and wherein the at least one layer (Lx) of polymer is made with silicone and it is comprised between two electrodes.

6. Stretchable counterpulsation tubular device according to one of claims 1 to 5, wherein the number of layers (Lx) is comprised between 2 and 50, forming a stack of layers, wherein each of the layers is comprised between two electrodes.

7. Stretchable counterpulsation tubular device according to one of the proceeding claims, wherein the at least one layer (Lx) has a thickness (Tx) of around 100 µm, a capacitance of around 1,6 nF and a resistance of around 1 kΩ and wherein the first voltage (V1) is around 6 kV, or wherein the at least one layer (Lx) has a thickness (Tx) of around 200 µm, a capacitance of around 0,8 nF and a resistance of 10 kΩ and wherein the first voltage (V1) is around 12 kV.

8. Stretchable counterpulsation tubular device according to one of claims 1 to 7, **characterized in that** it is adapted to surround or replace a portion of a tubular structure such as an artery.

9. Stretchable counterpulsation tubular device according to one of claims 1 to 8, **characterized in that** it further comprises a sensor adapted to detect or determine an increase of the internal pressure (P) of a flowing liquid (B) passing through the tubular device.

10. Stretchable counterpulsation tubular device according to one of claims 1 to 9, **characterized in that** it further comprises a shielding layer (Ls).

11. Stretchable counterpulsation tubular device according to one of the claims 1 to 10, **characterized in that** it further comprises a control unit (4), connected to the power supply (2), adapted to apply a given level of voltage at determined times and for determined periods of time and/or by intermittence.

12. Kit of several stretchable counterpulsation tubular devices, adapted for passively mimic the natural properties of an artery through which blood (B) flows, as described through claims 1 to 11, said kit comprising a first stretchable tubular device (1) having a natural force (F0) and at least a first modified force (F1) and at least a second stretchable tubular device (1') having a natural force (F0') and at least a first modified force (F1'), wherein at least one of the natural force (F0') and the modified force (F1') of the second stretchable tubular device (1') differs from the natural force (F0) and the modified force (F1) of the first stretchable tubular device (1).

13. Process for producing the stretchable counterpulsation tubular device adapted for passively mimic the natural properties of an artery through which blood (B) flows, as defined through claims 1 to 12, comprising step sequence of producing several combinations of a stretchable layers (Lx) and a conductive layer (Lc), and stacking said combinations so as to alternated the stretchable layers and conductive layers, resulting in a final stack of stretchable layers (SL), and rolling said final stack of stretchable layers (SL) around a tube (T) to produce a tubular shape, so that the diameter of said tubular shape of final stack of stretchable layers does not vary under the application of a voltage.

14. Process according to claim 13, further comprising a step of coating the obtained tubular shape with a conductive material to provide a shielding layer (Ls).

15. Stretchable counterpulsation tubular device, adapted for passively mimic the natural properties of an artery through which blood (B) flows, according to claim 11, wherein said control unit (4), is adapted to :
- apply at least one first voltage (V1) to the stretchable tubular device at a first instant (t1) , so as to induce a first modified force (F1) lower than its natural force (F0);
- apply at least one second voltage (V2) to the stretchable tubular device at a second instant (t2), so as to induce at least a second modified force (F2) different than the first modified force (F1);
wherein the first instant (t1) corresponds to the start of a first phase and the second instant (t2) corresponds to the start of a second phase and the end of the first phase, and wherein the first and the second phases are alternatively repeated.

16. Stretchable counterpulsation tubular device according to claim 15, wherein said stretchable tubular device is adapted to regulate the flowing of blood, and wherein said stretchable tubular device surrounds or replaces a portion of the aorta, said first phase corresponding to the systole and said second phase corresponding to the diastole, wherein during the first phase two intermediate voltages are alternatively applied several times or two or more constant voltages are successively applied.

17. Stretchable counterpulsation tubular device according to one of claims 15 and 16, wherein the voltage applied at the second instant (t2) or close to this second instant (t2) is null and remains null until the start of the first phase.

## Patentansprüche

1. Dehnbare röhrenförmige Gegenpulsationsvorrichtung (1), welche angepasst ist, die natürlichen Eigenschaften einer Arterie, durch welche Blut (B) fliesst, passiv zu imitieren, umfassend mindestens eine Schicht (Lx) aus einem dehnbaren Polymer, eine Stromversorgung (2) und einen Satz von Elektroden (3a, 3b), die mit der besagten Stromversorgung (2) verbunden sind, worin die Stromversorgung derart angepasst ist, um mindestens einen ersten Spannungspegel (V1) an die besagten Elektroden zu liefern, **dadurch gekennzeichnet, dass** die besagte röhrenförmige Vorrichtung einen natürlichen Durchmesser (D0) und eine inhärente Viskoelastizität (E0) aufweist, welche eine natürliche Kraft (F0) ausübt, die auf seinen Innenraum (Si) gerichtet ist, wenn sein Durchmesser durch die ausströmende Kraft des Blutes auf einen Durchmesser (D1) ausgedehnt wird, der grösser als sein natürlicher Durchmesser (D0) ist, und dass es mindestens einen ersten Pegel modifizierter Viskoelastizität (E1) aufweist, wenn ein erster Spannungspegel (V1) an die Schichten (Lx) aus dehnbarem Polymer durch die Elektroden (3a, 3b) geliefert wird, wobei der besagte erste Pegel modifizierter Viskoelastizität eine erste modifizierte Kraft (F1) ausübt, die auf den Innenraum der röhrenförmigen Vorrichtung gerichtet ist, wenn sein Durchmesser durch die ausströmende Kraft des Blutes auf einen Durchmesser (D1) ausgedehnt wird, der grösser als sein natürlicher Durchmesser (D0) ist, wobei die erste modifizierte Kraft (F1) niedriger als die natürliche Kraft (F0) ist.

2. Dehnbare röhrenförmige Gegenpulsationsvorrichtung gemäss Anspruch 1, worin die besagte mindestens eine erste Spannung (V1) keinen Einfluss auf den Durchmesser der dehnbaren rohrförmigen Vorrichtung hat.

3. Dehnbare röhrenförmige Gegenpulsationsvorrichtung gemäss einem der Ansprüche 1 und 2, worin die besagte dehnbare röhrenförmige Gegenpulsationsvorrichtung frei von mechanischen Teilen wie Federn und Ballons ist.

4. Dehnbare röhrenförmige Gegenpulsationsvorrichtung gemäss Ansprüchen 1 bis 3, worin die Dicke (Tx) der mindestens einen Schicht (Lx) zwischen etwa 10 µm und etwa 500 µm liegt, beispielsweise etwa 50 µm, 100 µm, 200 µm oder 300 µm beträgt.

5. Dehnbare röhrenförmige Gegenpulsationsvorrichtung gemäss Anspruch 1 bis 4, worin die Elektroden (3a, 3b) aus Kohlenstoff bestehen, und worin die mindestens eine Polymerschicht (Lx) aus Silikon besteht und zwischen zwei Elektroden angeordnet ist.

6. Dehnbare röhrenförmige Gegenpulsationsvorrichtung gemäss einem der Ansprüche 1 bis 5, worin die Anzahl der Schichten (Lx) zwischen 2 und 50 liegt und einen Schichtenstapel bildet, wobei jede der Schichten zwischen zwei Elektroden angeordnet ist.

7. Dehnbare röhrenförmige Gegenpulsationsvorrichtung gemäss einem der vorhergehenden Ansprüche, worin die mindestens eine Schicht (Lx) eine Dicke (Tx) von etwa 100 µm, eine Kapazität von etwa 1,6 nF und einen Widerstand von etwa 1 kΩ aufweist, und worin die erste Spannung (V1) etwa 6 kV beträgt, oder worin die mindestens eine Schicht (Lx) eine Dicke (Tx) von etwa 200 µm, eine Kapazität von etwa 0,8 nF und einen Widerstand von 10 kΩ aufweist, und worin die erste Spannung (V1) etwa 12 kV beträgt.

8. Dehnbare röhrenförmige Gegenpulsationsvorrichtung gemäss einem der Ansprüche 1 bis 7, **dadurch gekennzeichnet, dass** es dazu angepasst ist, einen Abschnitt einer röhrenförmigen Struktur wie einer Arterie zu umgeben oder zu ersetzen.

9. Dehnbare röhrenförmige Gegenpulsationsvorrichtung gemäss einem der Ansprüche 1 bis 8, **dadurch gekennzeichnet, dass** es zudem einen Sensor umfasst, der dazu geeignet ist, einen Anstieg des Innendrucks (P) einer durch die röhrenförmige Vorrichtung fliessenden Flüssigkeit (B) zu erkennen oder festzustellen.

10. Dehnbare röhrenförmige Gegenpulsationsvorrichtung gemäss einem der Ansprüche 1 bis 9, **dadurch gekennzeichnet, dass** sie zudem eine Abschirmschicht (Ls) umfasst.

11. Dehnbare röhrenförmige Gegenpulsationsvorrichtung gemäss einem der Ansprüche 1 bis 10, **dadurch gekennzeichnet, dass** sie ausserdem eine Steuereinheit (4) umfasst, welche an die Stromversorgung (2) angeschlossen und dazu angepasst ist, zu festgelegten Zeitpunkten und für festgelegte Zeiträume und/oder mit Unterbrechungen eine bestimmte Spannung anzulegen.

12. Kit aus mehreren dehnbaren röhrenförmigen Gegenpulsationsvorrichtungen, welche angepasst sind, die natürlichen Eigenschaften einer Arterie, durch welche Blut (B) fliesst, passiv zu imitieren, wie in den Ansprüchen 1 bis 11 beschrieben, wobei das besagte Kit eine erste dehnbare röhrenförmige Vorrichtung (1) mit einer natürlichen Kraft (F0) und mindestens einer ersten modifizierten Kraft (F1), und mindestens eine zweite dehnbare röhrenförmige Vorrichtung (1') mit einer natürlichen Kraft (F0') und mindestens einer ersten modifizierten Kraft (F1') umfasst, worin sich mindestens eine der natürlichen Kraft (F0') und der modifizierten Kraft (F1') der zweiten dehnbaren röhrenförmigen Vorrichtung (1') von der natürlichen Kraft (F0) und der modifizierten Kraft (F1) der ersten dehnbaren röhrenförmigen Vorrichtung (1) unterscheidet.

13. Verfahren zur Herstellung der dehnbaren röhrenförmigen Gegenpulsationsvorrichtung, welche angepasst ist, die natürlichen Eigenschaften einer Arterie, durch welche Blut (B) fliesst, passiv zu imitieren, wie in den Ansprüchen 1 bis 12 definiert, umfassend die Schrittfolge der Herstellung mehrerer Kombinationen aus dehnbaren Schichten (Lx) und einer leitfähigen Schicht (Lc) und des Stapelns der besagten Kombinationen, so dass sich die dehnbaren Schichten und die leitfähigen Schichten abwechseln, was zu einem endgültigen Stapel dehnbarer Schichten (SL) führt, und des Rollens dieses endgültigen Stapels dehnbarer Schichten (SL) um eine Röhre (T), um eine Röhrenform zu erzeugen, so dass sich der Durchmesser der besagten röhrenförmigen Form des endgültigen Stapels dehnbarer Schichten unter Anlegen einer Spannung nicht ändert.

14. Verfahren gemäss Anspruch 13, zudem umfassend einen Schritt des Beschichtens der erhaltenen röhrenförmigen Form mit einem leitfähigen Material, um eine Abschirmschicht (Ls) zu schaffen.

15. Dehnbare röhrenförmige Gegenpulsationsvorrichtung, welche angepasst ist, die natürlichen Eigenschaften einer Arterie, durch welche Blut (B) fliesst, passiv zu imitieren, gemäss Anspruch 11, worin die besagte Steuereinheit (4) dazu angepasst ist:
- mindestens eine erste Spannung (V1) an die dehnbare röhrenförmige Vorrichtung zu einem ersten Zeitpunkt (t1) anzulegen, um eine erste modifizierte Kraft (F1) zu induzieren, die niedriger als ihre natürliche Kraft (F0) ist;
- mindestens eine zweite Spannung (V2) an die dehnbare röhrenförmige Vorrichtung zu einem zweiten Zeitpunkt (t2) anzulegen, um mindestens eine zweite modifizierte Kraft (F2) zu induzieren, die sich von der ersten modifizierten Kraft (F1) unterscheidet;
worin der erste Zeitpunkt (t1) dem Beginn einer ersten Phase und der zweite Zeitpunkt (t2) dem Beginn einer zweiten Phase und dem Ende der ersten Phase entspricht, und worin die erste und die zweite Phase abwechselnd wiederholt werden.

16. Dehnbare röhrenförmige Gegenpulsationsvorrichtung gemäss Anspruch 15, worin die besagte dehnbare röhrenförmige Vorrichtung zur Regulierung des Blutflusses angepasst ist, und worin die besagte dehnbare röhrenförmige Vorrichtung einen Teil der Aorta umgibt oder ersetzt, wobei die besagte erste Phase der Systole und die besagte zweite Phase der Diastole entspricht, worin während der ersten Phase zwei Zwischenspannungen mehrmals abwechselnd angelegt werden oder zwei oder mehr konstante Spannungen nacheinander angelegt werden.

17. Dehnbare röhrenförmige Gegenpulsationsvorrichtung gemäss einem der Ansprüche 15 und 16, worin die zum zweiten Zeitpunkt (t2) oder nahe diesem zweiten Zeitpunkt (t2) angelegte Spannung Null ist und bis zum Beginn der ersten Phase Null bleibt.

## Revendications

1. Dispositif tubulaire extensible de contre-pulsation (1) adapté pour imiter passivement les propriétés naturelles d'une artère à travers laquelle circule le sang (B), comprenant au moins une couche (Lx) d'un polymère extensible, une alimentation électrique (2) et un ensemble d'électrodes (3a, 3b) connectées à ladite alimentation électrique (2), dans lequel l'alimentation électrique est adaptée pour fournir au moins un premier niveau de tension (V1) auxdites électrodes, **caractérisé en ce que** ledit dispositif tubulaire de contre-pulsation a un diamètre natif (D0) et une viscoélasticité inhérente (E0) exerçant une force naturelle (F0) dirigée vers son espace intérieur (Si) lorsque son diamètre est étendu à un diamètre (D1) par la force d'expansion du sang supérieure à son diamètre natif (D0), et **en ce qu'**il présente au moins un premier niveau de viscoélasticité modifiée (E1) lorsqu'un premier niveau de tension (V1) est fourni aux couches (Lx) de polymère extensible par l'intermédiaire des électrodes (3a, 3b), ledit premier niveau de viscoélasticité modifiée exerçant une première force modifiée (F1) dirigée vers l'espace interne du dispositif tubulaire lorsque son diamètre est étendu à un diamètre (D1) par la force d'expansion du sang supérieure à son diamètre initial (D0), la première force modifiée (F1) étant inférieure à la force naturelle (F0).

2. Dispositif tubulaire extensible à contre-pulsation selon la revendication 1, dans lequel ladite au moins une première tension (V1) n'a aucune incidence sur le diamètre du dispositif tubulaire extensible.

3. Dispositif tubulaire extensible à contre-pulsation selon l'une des revendications 1 et 2, dans lequel ledit dispositif tubulaire extensible à contre-pulsation est exempt de pièces mécaniques telles que des ressorts et des ballons.

4. Dispositif tubulaire extensible à contre-pulsation selon les revendications 1 à 3, dans lequel l'épaisseur (Tx) de la au moins une couche (Lx) est comprise entre environ 10 µm et environ 500 µm, telle qu'environ 50 µm, 100 µm, 200 µm ou 300 µm.

5. Dispositif tubulaire extensible à contre-pulsation selon les revendications 1 à 4, dans lequel les électrodes (3a, 3b) sont en carbone, et dans lequel la au moins une couche (Lx) de polymère est en silicone et est comprise entre deux électrodes.

6. Dispositif tubulaire extensible de contre-pulsation selon l'une des revendications 1 à 5, dans lequel le nombre de couches (Lx) est compris entre 2 et 50, formant un empilement de couches, dans lequel chacune des couches est comprise entre deux électrodes.

7. Dispositif tubulaire extensible à contre-pulsation selon l'une des revendications précédentes, dans lequel la au moins une couche (Lx) a une épaisseur (Tx) d'environ 100 µm, une capacité d'environ 1,6 nF et une résistance d'environ 1 kΩ et dans lequel la première tension (V1) est d'environ 6 kV, ou dans lequel la au moins une couche (Lx) a une épaisseur (Tx) d'environ 200 µm, une capacité d'environ 0,8 nF et une résistance de 10 kΩ et dans lequel la première tension (V1) est d'environ 12 kV.

8. Dispositif tubulaire extensible à contre-pulsation selon l'une des revendications 1 à 7, **caractérisé en ce qu'**il est adapté pour entourer ou remplacer une partie d'une structure tubulaire telle qu'une artère.

9. Dispositif tubulaire extensible de contre-pulsation selon l'une des revendications 1 à 8, **caractérisé en ce qu'**il comprend en outre un capteur adapté pour détecter ou déterminer une augmentation de la pression interne (P) d'un liquide (B) s'écoulant à travers le dispositif tubulaire.

10. Dispositif tubulaire extensible de contre-pulsation selon l'une des revendications 1 à 9, **caractérisé en ce qu'**il comprend en outre une couche écran (Ls).

11. Dispositif tubulaire de contre-pulsation extensible selon l'une des revendications 1 à 10, **caractérisé en ce qu'**il comprend en outre une unité de commande (4), reliée à l'alimentation électrique (2), adaptée pour appliquer un niveau de tension donné à des moments déterminés et pendant des périodes déterminées et/ou de manière intermittente.

12. Kit de plusieurs dispositifs tubulaires de contre-pulsation extensibles, adaptés pour imiter passivement les propriétés naturelles d'une artère à travers laquelle s'écoule du sang (B), tel que décrit dans les revendications 1 à 11, ledit kit comprenant un premier dispositif tubulaire extensible (1) ayant une force naturelle (F0) et au moins une première force modifiée (F1) et au moins un deuxième dispositif tubulaire extensible (1') ayant une force naturelle (F0') et au moins une première force modifiée (F1'), dans lequel au moins l'une de la force naturelle (F0') et de la force modifiée (F1') du deuxième dispositif tubulaire extensible (1') diffère de la force naturelle (F0) et de la force modifiée (F1) du premier dispositif tubulaire extensible (1).

13. Procédé de fabrication du dispositif tubulaire extensible à contre-pulsation adapté pour imiter passivement les propriétés naturelles d'une artère à travers laquelle s'écoule du sang (B), tel que défini par les revendications 1 à 12, comprenant une séquence d'étapes consistant à produire plusieurs combinaisons d'une couche extensible (Lx) et d'une couche conductrice (Lc), et d'empiler lesdites combinaisons de manière à alterner les couches extensibles et les couches conductrices, ce qui donne un empilement final de couches extensibles (SL), et de rouler ledit empilement final de couches extensibles (SL) autour d'un tube (T) pour produire une forme tubulaire, de telle sorte que le diamètre de ladite forme tubulaire de l'empilement final de couches extensibles ne varie pas sous l'application d'une tension.

14. Procédé selon la revendication 13, comprenant en outre une étape consistant à revêtir la forme tubulaire obtenue d'un matériau conducteur afin de fournir une couche écran (Ls).

15. Dispositif tubulaire de contre-pulsation extensible, adapté pour imiter passivement les propriétés naturelles d'une artère à travers laquelle s'écoule du sang (B), selon la revendication 11, dans lequel ladite unité de commande (4) est adaptée pour :
- appliquer au moins une première tension (V1) au dispositif tubulaire extensible à un premier instant (t1), de manière à induire une première force modifiée (F1) inférieure à sa force naturelle (F0) ;
- appliquer au moins une deuxième tension (V2) au dispositif tubulaire extensible à un deuxième instant (t2), de manière à induire au moins une deuxième force modifiée (F2) différente de la première force modifiée (F1) ;
dans lequel le premier instant (t1) correspond au début d'une première phase et le deuxième instant (t2) correspond au début d'une deuxième phase et à la fin de la première phase, et dans lequel les première et deuxième phases sont répétées en alternance.

16. Dispositif tubulaire extensible à contre-pulsation selon la revendication 15, dans lequel ledit dispositif tubulaire extensible est adapté pour réguler le flux sanguin, et dans lequel ledit dispositif tubulaire extensible entoure ou remplace une partie de l'aorte, ladite première phase correspondant à la systole et ladite deuxième phase correspondant à la diastole, dans lequel, pendant la première phase, deux tensions intermédiaires sont appliquées alternativement plusieurs fois ou deux tensions constantes ou plus sont appliquées successivement.

17. Dispositif tubulaire extensible de contre-pulsation selon l'une des revendications 15 et 16, dans lequel la tension appliquée au deuxième instant (t2) ou à proximité de ce deuxième instant (t2) est nulle et reste nulle jusqu'au début de la première phase.
